# EUROPEAN PATENT SPECIFICATION

(11) **EP 3 980 101 B1**
(45) Date of publication and mention of the grant of the patent: **18.12.2024**
(21) Application number: 19932111.8
(22) Date of filing: 06.06.2019
(51) Int. Cl.: A61M 16/00, A61M 16/10, A61M 16/12

(54) **NOISE REDUCTION DEVICE FOR RESPIRATORY APPARATUS**
GERÄUSCHVERMINDERUNGSVORRICHTUNG FÜR BEATMUNGSEINRICHTUNG
DISPOSITIF DE RÉDUCTION DE BRUIT POUR APPAREIL RESPIRATOIRE

(43) Date of publication of application: 13.04.2022
(73) Proprietor: Vincent Medical (Dong Guan) Manufacturing Co., Ltd., Dongguan, Guangdong 523730 (CN); Vincent Medical (Dong Guan) Technology Co., Ltd, Dongguan, Guangdong 523808 (CN)
(72) Inventor: XU, Jiebing, Dongguan, Guangdong 523730 (CN); YU, Haibin, Dongguan, Guangdong 523730 (CN); HU, Zhenxiang, Dongguan, Guangdong 523730 (CN); LEI, Yu, Shenzhen, Guangdong 518109 (CN)
(74) Representative: Pfenning, Meinig & Partner mbB
(86) International application number: PCT/CN2019/090387
(87) International publication number: WO 2020/243956

(56) References cited:
- CN-A- 101 075 431
- CN-C- 100 587 804
- CN-U- 202 105 279
- CN-U- 204 025 176
- CN-U- 204 025 176
- CN-U- 206 613 010
- CN-U- 206 613 010
- CN-U- 207 323 803
- JP-A- 2001 278 604
- US-A1- 2012 037 160
- US-A1- 2015 273 167

## Description

### TECHNICAL FIELD

The present invention relates to a noise reduction device for a respiratory apparatus, particularly, for noise reduction at a gas inlet of the respiratory apparatus.

### BACKGROUND

In modern clinical medicine, a respiratory apparatus is commonly used for patients with respiratory illnesses such as acute respiratory distress syndrome, severe asthma and chronic obstructive pulmonary disease, as well as used for anesthesia and respiratory management during surgery, first aid resuscitation, and even domestic use for supportive treatment. A respiratory apparatus is a vital medical device that can prevent and treat respiratory failure, reduce complications and prolong the patient's life.

Current respiratory apparatuses have a number of drawbacks. For example, when air is drawn into a respiratory apparatus by a blower, noise is generated by the friction between the air flow and the gas inlet passage. The noise is particularly obvious when the respiratory apparatus is used in a quiet environment or when the patient is sleeping, potentially causing a physical and mental annoyance to the patient.

There are many designs of a noise reduction device to reduce the noise generated at the gas inlet of the respiratory apparatus. For example, CN101075431A to Hongqing Wang, published on 21 November 2007, discloses a noise reduction device including a gas passage defined by a side wall to direct gas flow into the respiratory apparatus. The gas may only travel an angular distance of 60 degrees before reaching the gas outlet, which is too short for the gas passage to reduce the turbulent gas flow and thus provide an effective noise reduction. Accordingly, it has been found that such a design cannot provide significant noise reduction.

It is therefore desirable to provide an improved noise reduction device for the gas to flow from the gas inlet to the gas outlet in order to reduce noise level at the gas inlet of a respiratory apparatus significantly.

CN100587804C discloses a silencing device including a main body and an upper cover, and the upper cover is buckled on the main body through a flange provided at the edge of the cover to form a planar spiral air passage arranged in the silencing device. The spiral air passage includes an air inlet arranged at the outer end of the spiral air passage, and an air outlet disposed at the center of the inner end of the spiral air passage and perpendicular to the spiral plane of the spiral air passage.

CN204025176U discloses a silencing device, which includes an upper cover, a middle body and a base. The middle body is located between the upper cover and the base. The upper cover is provided with a spiral stereoscopic rotary air inlet integral with the upper cover.

CN206613010U provides a silencing cover, including a cover body, with a planar spiral air passage disposed therein and an air passage outlet disposed thereon, and the planar spiral air passage forms an air passage inlet along the circumferential direction of the cover body.

JP2001278604A provides an oxygen enricher of which the noise caused by cooling air for a motor and the like and adsorbed nitrogen exhaust is decreased effectively by that: a guide is installed in the exhaust silencing chamber to rotate the exhaust gas introduced from the exhaust inlet and to exhaust the gas from the exhaust outlet to the outside.

### SUMMARY

The invention is defined in the appended claims. The present disclosure provides a noise reduction device for a respiratory apparatus to, at least, solve the technical problem of the noise generated by fat the gas inlet of the current respiratory apparatus.

According to an aspect of the present disclosure, there is provided a noise reduction device for a respiratory apparatus including a body configured to be mounted on the gas inlet of the respiratory apparatus, and a cover configured to be detachably engageable with the body for forming a gas inlet and a gas passage. The body includes a side wall and a gas outlet and the cover includes a guiding member defining at least a part of the gas passage. The guiding member is configured to be coupled with the side wall of the body to form the gas passage between the body and the cover. The gas passage formed directs a flow of gas to an angular rotation about a centre of the gas outlet of at least 180 degrees to 330 degrees relative to the gas inlet before discharging at the gas outlet. The noise reduction device further includes: a locking mechanism for slidably locking the cover to the body. The locking mechanism includes at least two slots arranged on the body, and at least two corresponding tabs arranged on the cover. The cover is engaged with the body for forming the gas inlet and the gas passage by that the at least two corresponding tabs arranged on the cover are slidably locked to the at least two slots arranged on the body respectively.

According to another aspect of the disclosure, there is provided a respiratory apparatus including the noise reduction device substantially as described herein. The respiratory apparatus may further include a pressurized gas inlet for supplying a pressurized gas; a chamber in fluid communication with the gas outlet of the noise reduction device and the pressurized gas inlet for mixing atmospheric air and the pressurized gas; and a noise-damping device disposed downstream of the chamber.

Without intending to be limited by theory, it is believed that the noise reduction device in the present disclosure provides significant advantages over, for example, current noise reduction devices that only allow a gas flow to turn about 60 degrees relative to the gas inlet before discharging at the gas outlet. Specifically, it is believed that the noise reduction device in the present disclosure may provide a longer path, and in an embodiment herein, a longer spiral passage for the gas to flow from the gas inlet to the gas outlet so as to decrease the turbulence and resistance of gas flow to a larger extent and hence reducing the noise caused by the friction between the turbulent gas flow and the gas inlet of the respiratory apparatus. Moreover, it is believed that the configuration of the guiding member being located on the cover provides an easier and more convenient way to clean the gas passage. The cover can be disengaged from the body and subject to common sterilization methods of medical equipment. Such arrangement may also facilitate replacement of the cover in case abrasion or damage is found on the guiding member which may increase turbulent flow of the incoming gas and thus causes noise.

### BRIEF DESCRIPTION OF DRAWINGS

Figure 1 shows a noise reduction device in the reference CN101075431A (see above);
Figure 2 shows a perspective view of an embodiment of the noise reduction device herein when the body and the cover are disengaged from each other;
Figure 3 shows a front view of an embodiment of the body before engaging with the cover;
Figure 4 shows a rear view of the embodiment of the cover of Figure 2;
Figure 5 shows an embodiment of the noise reduction device when the body and the cover are engaged;
Figure 6 shows the direction of the gas flow in an embodiment of the noise reduction device when the body is engaged with the cover;
Figure 7 shows an embodiment of the noise reduction device being mounted to a respiratory apparatus; and
Figure 8 shows an embodiment of the components of the respiratory apparatus in Figure 7.

The figures herein are for illustrative purposes only and are not necessarily drawn to scale.

### DETAILED DESCRIPTION

The present disclosure relates to a noise reduction device which is useful to minimize the noise generated when a gas, in particular atmospheric air or pressurized gas, enters the associated apparatus such as, but is not limited to, a respiratory apparatus which requires a supply of a gas. The respiratory apparatus may be, but is not limited to, a humidifier, a respirator, a nebulizer, etc.

The gas useful herein typically includes atmospheric air or air enriched with oxygen gas, as desired. The gas herein may be at ambient room temperature, higher than room temperature, or lower that room temperature, as desired. The gas herein may be at the ambient pressure of the surrounding environment, or at a higher pressure than the surrounding environment. The gas herein may be at ambient humidity, more humid than ambient humidity, or drier than ambient humidity, as desired.

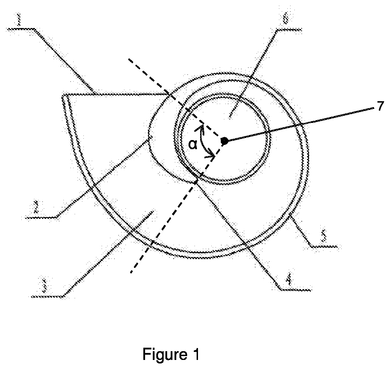

Figure 1 shows a noise reduction device disclosed in, for example, CN101075431A (see above). The noise reduction device includes a gas passage 3 defined by a side wall 5. A gas inlet 1 is provided at outer end of the gas passage 3 and a gas outlet 6 with a centre 7 is provided at the inner end of the gas passage 3. Two dashed lines are added to Figure 1, with one extending from the centre 7 to the gas inlet 1 and one extending from the centre 7 to an inner end 4 of the side wall 5, to define an angular rotation αabout the centre 7 to show the shortest distance for the incoming gas to travel from the gas inlet 1 to the gas outlet 6, which is about 90 degrees. In other words, gas may only travel about 90 degrees before reaching the gas outlet, which is too short for the gas passage to reduce the turbulent gas flow and thus provide an effective noise reduction. Accordingly, it has been found that such a design cannot provide significant noise reduction.

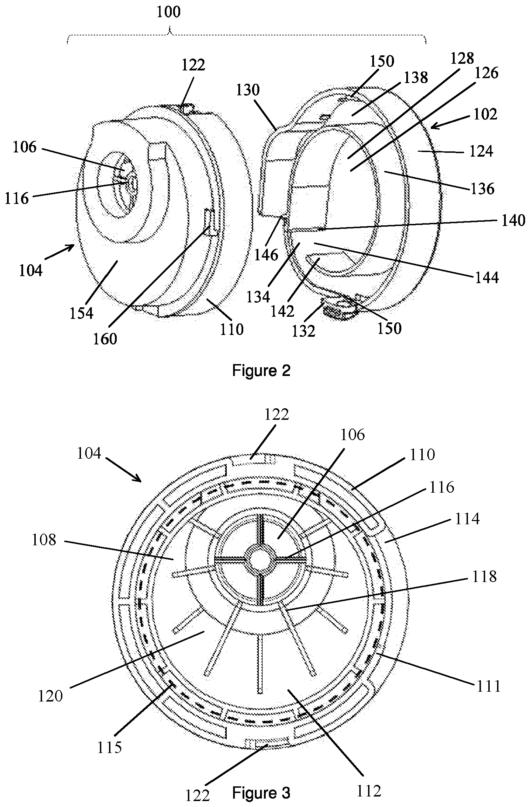

Referring to Figure 2, there is illustrated an embodiment of a noise reduction device of the present disclosure. The noise reduction device 100 of the present disclosure has a cover 102 and a body 104. The cover 102 and the body 104 are, preferably, separately manufactured and can be detachably engaged with each other through a locking means such as sliding and screwing.

In this embodiment, the cover 102 and the body 104 may be made of a plastic, such as a thermoset plastic, a resin, a polymeric material, etc. Such plastics are known in the art and typically include materials such as polycarbonate, polyethylene, polypropylene, polyvinyl chloride, acrylonitrile butadiene styrene, polymethyl methacrylate, phenolics, melamine formaldehyde, polysulfone, polyetherimide, polyethylene terephthalate, urea-formaldehyde, polyether ether ketone, and a combination thereof. Furthermore, the plastic may incorporate an anti-microbial compound by, for example, containing a coating, integrating the anti-microbial compound into the plastic, etc.

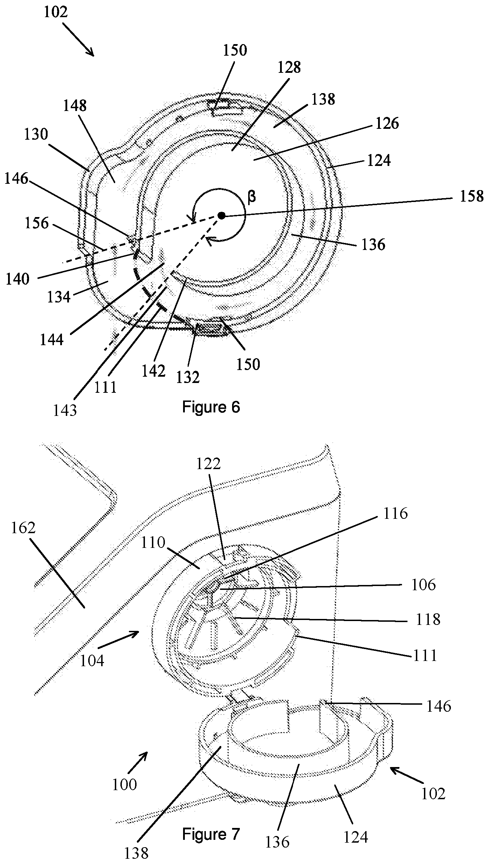

Figure 3 shows an embodiment of the body 104 before engaging with the cover (see Figure 2 at 102) . The body 104 has a gas outlet 106 and is preferably arranged to be mounted to a respiratory apparatus (see Figure 7 at 162) so as to discharge a gas into the respiratory apparatus for subsequent use. In this embodiment, the body 104 is configured with a cavity 108 surrounded by a side wall 110. The side wall 110 extends perpendicularly from periphery of an inner surface 112 and includes a first end portion 111. The cavity 108 may be open or closed depending on the configuration of the side wall 110. In this embodiment, the cavity 108 is open with the side wall 110 configured as a C-shape, i.e. leaving an open portion 114. At least part of the side wall 110 can be coupled to the cover (see Figure 2 at 102) for forming a tight seal.

The cavity 108 may house a filter 115 (shown as a dotted line) therein. The filter 115 may be provided to filter dust, pollen, mold, bacteria, etc. from the gas, particularly atmospheric air, before the gas enters the respiratory apparatus. In an embodiment where the filter 115 is detachably arranged in the cavity 108 of the body 104, the filter 115 can be replaced with a new one either randomly or regularly so as to keep the filtered gas free from, or at least with a reduced amount of, dust, pollen, mold, bacteria, etc. This is particularly advantageous when the respiratory apparatus is used for clinical applications. It is also believed that the filter 115 can also act as a noise suppressor to reduce the noise generated in the cavity 108 when the gas passes through the noise reduction device (see Figure 2 at 100). The filter 115 may be, for example, a paper filter, foam filter, cotton filter, or high-efficiency particulate air filter. One skilled in the art would appreciate that various suitable filters can be applied to the noise reduction device 100 of the present disclosure.

In this embodiment, the gas outlet 106 is radially offset and is supported by a supporting structure 116 which has a plurality of upright protrusions 118 on the inner surface 112 connecting to the gas outlet 106. The gas outlet 106 may be aligned with the gas pathway in the respiratory apparatus, thereby reducing the formation of turbulence. One skilled in the art would appreciate that the gas outlet 106 may be positioned at the centre of the cavity 108 to achieve the similar purpose.

The cavity 108 may further include a converging portion 120 on the inner surface 112 which converges towards the gas outlet 106 so as to facilitate the gas flow. In addition to guiding the flow of the gas towards the gas outlet 106, the supporting structure 116 may also help to hold the filter 115 in place. Without intending to be limited by theory, it is also believed that the upright protrusions 118 and supporting structure 116 may further enhance the structural integrity of the body 104 and/or the cover (see Figure 2 at 102). The upright protrusions 118 and the converging portion 120 support the filter 115 which may help to separate the filter 115 from the inner surface 112 to increase the effective surface area of the filter 115 and hence increase the amount of filtered gas flow. This may synergistically help to protect a blower of the respiratory apparatus (see Figure 7 at 162) by reducing its workload and thus further reducing the noise produced. In this embodiment, the upright protrusions 118 of the supporting structure 116 are configured as extending, continuously or discontinuously, radially from the gas outlet 106.

In the embodiment of Figures 2 and 3, the body 104 is configured to detachably engage with the cover 102. Preferably, the body 104 is enclosed by the cover 102 after engaging with the cover 102. The body 104 may include two slots 122 (or tabs) respectively arranged on substantially diametrically opposite sides of the side wall 110 for complementary slide locking with corresponding tabs (or slots) on the cover 102.

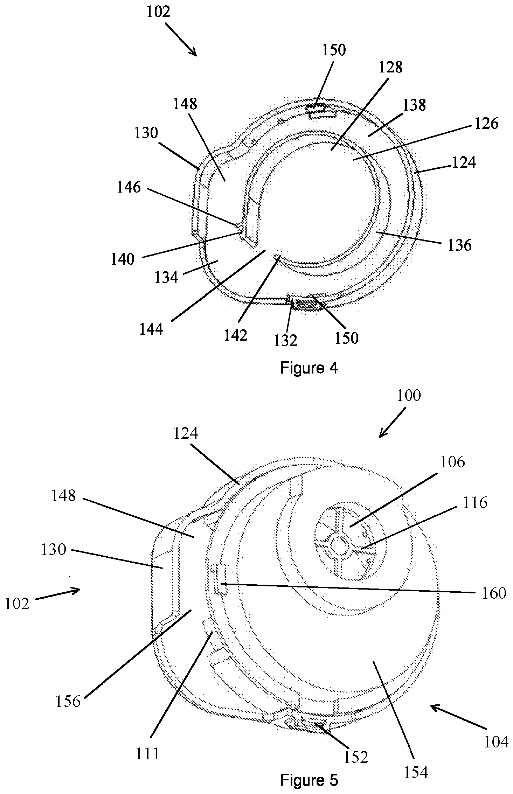

Turning to the cover 102, with reference to Figure 4 showing a rear view of it, the cover 102 has a side wall 124 and an inner surface 126 facing towards the inner surface (see Figure 3 at 112) of the body (see Figure 3 at 104) when it is engaged with the body (see Figure 3 at 104) to form the noise reduction device (see Figure 2 at 100). The side wall 124 extends perpendicularly from the periphery of the inner surface 126 and partially surrounds the cover 102 to form a cavity 128. In this embodiment, the cavity 128 is open with the side wall 124 configured substantially as a C-shape to define a second end portion 130 and a third end portion 132 on the side wall 124 respectively, leaving an open portion 134.

The cover 102 has a guiding member 136 being configured to extend substantially perpendicularly from the inner surface 126. The guiding member 136 itself defines at least a part of a gas passage 138, and is configured in a way to form the gas passage 138 between the body (see Figure 3 at 104) and the cover 102 when they are engaged together. One skilled in the art would appreciate that possible configurations of the guiding member such as a spiral including Cotes's spiral, Archimedean spiral and golden spiral, may be used depending on the desired design and noise reduction requirements. Preferably, the area enclosed by the guiding member 136 is at least twice than area of the gas outlet 106 in order to increase the effective filtering area of the filter 115 and reduce gas resistance, thereby further reducing noise production.

In this embodiment, the guiding member 136 is substantially in form of a C-shape. The guiding member 136 has a fourth end portion 140 and a fifth end portion 142 defining an opening 144 aligning with the open portion 134 and to be closed by the side wall 110 of the body (see Figure 3 at 104) when the body (see Figure 3 at 104) and the cover 102 are engaged. The fourth end portion 140 includes a projection 146 for additional engagement and position fixing with the first end portion 111 of the body 104 (see Figure 3 at 111) when the body 104 and the cover 102 are engaged together.

The fourth end portion 140 and the second end portion 130 together define a flow deflecting portion 148 being a part of the gas passage 138 to provide an enlarged section for an increased level of gas entry, and facilitate a spiral flow of the gas into the gas passage. The flow deflecting portion 148 may also avoid transmission of noise from the blower inside the respiratory apparatus to the outside environment.

In this embodiment, the cover 102 is detachably engageable with the body (see Figure 3 at 104) and preferably encloses the body 104 after engagement. Similar to the body 104, two tabs 150 may be respectively arranged on substantially diametrically opposite sides of the side wall 124 for complementary slide locking with corresponding slots 122 on the body 104 to form a bayonet mount.

Figure 5 shows the noise reduction device 100 when the body 104 and the cover 102 are slidably locked to one another. In this embodiment, the body 104 is oriented and inserted into the cavity (see Figure 4 at 128) of the cover 102 with the tabs (see Figure 4 at 150) being received in the slots (see Figure 3 at 122). A slight turning of either the body 104 or the cover 102 locks the two components with a bayonet lock as a locking mechanism 152 to hold them in place. In the present disclosure, the locking mechanism 152 includes at least one slot (see Figure 3 at 122) arranged on the body 104, and at least one corresponding tab (see Figure 4 at 150) arranged on the cover 102. In another embodiment, the locking mechanism 152 may include a pair of magnetic members arranged on the cover 102 and the body 104 as the locking means. One skilled in the art would appreciate that other locking means, such as a push lock, a slide lock, a screw, a plug and/or a combination thereof, may be used herein.

In this figure, the outer surface 154 of the body 104 shows the gas outlet 106 which is to be mounted to a respiratory apparatus (see Figure 7 at 162) for discharge of a gas into the respiratory apparatus. The flow deflecting portion 148, which is not covered by the body 104, is shown adjacent to the second end portion 130 of the side wall 124. Adjacent to the flow deflecting portion 148 is a gas inlet 156 arranged between the side wall (see Figure 3 at 110) and the side wall 124. The first end portion 111 of the side wall 110 is arranged adjacent to the gas inlet 156. Two tabs 160 (only one is shown) are disposed on substantially diametrically opposite ends of the outer surface 154 for detachable mounting on the respiratory apparatus (see Figure 7 at 162) through sliding. One skilled in the art would appreciate that other locking means such as screwing may also be used depending on the configuration of the respiratory apparatus.

Referring to Figure 6, when the body (see Figure 2 at 104) and the cover 102 are engaged, the side wall 110 of the body (see Figure 2 at 104) is received in the gas passage 138 and the body (see Figure 2 at 104) is enclosed by the cover 102. The location of the first end portion (see Figure 3 at 111) is shown as a dotted line forming a seal with the projection 146 and abutting the fourth end portion 140 of the guiding member 136 to define the planar spiral gas passage 138 between the side wall 110 and the guiding member 136, wherein the fifth end portion 142 spaces apart from the side wall (see Figure 3 at 110) to form a gap 143 and the gas inlet 156 is arranged to be perpendicularly to the gas outlet 106 in this embodiment. In an embodiment where a filter (see Figure 3 at 115) is placed in the body (see Figure 3 at 104), the guiding member 136 is in contact with the filter (see Figure 3 at 115) when the body (see Figure 3 at 104) and the cover 102 are engaged so as to keep the filter (see Figure 3 at 115) in place by sandwiching the filter (see Figure 3 at 115) between the guiding member 136 and the body (see Figure 2 at 104). This also helps to avoid oscillation of the filter (see Figure 3 at 115) between the body (see Figure 3 at 104) and the cover 102 when the gas passes the filter (see Figure 3 at 115).

During operation, a gas, typically atmospheric air, is drawn to the gas inlet 156 preferably by a blower of the respiratory apparatus, where the gas travels from the flow deflecting portion 148 of a wider cross section to the gas passage 138 of a narrower cross section for a smoother gas flow by maintaining or even reducing gas resistance. The gas then flows through the gas passage 138, the gap 143, and to the opening 144. The gas then passes through the filter (see Figure 3 at 115) which is in contact with the guiding member 136 when the body (see Figure 3 at 104) and the cover 102 are engaged, and finally reaches the gas outlet (see Figure 3 at 106) (shown by arrows). One skilled in the art would appreciate that with such configuration, the incoming gas is forced to travel an angular rotation β about a centre 158 of the gas outlet (see Figure 3 at 106) of at least 330 degrees from the gas inlet 156 to the gas outlet 106, which is over 2.5 times longer than the gas passage 3 described in Figure 1 without substantive increment in size of the noise reduction device 100. In an alternative embodiment, the gas passage 138 formed may direct the gas flow to travel an angular rotation β about the centre 158 of the gas outlet (see Figure 3 at 106) of at least 180 degrees, at least 270 degrees or at least 300 degrees, relative to the gas inlet 156 before discharging at the gas outlet (see Figure 3 at 106).

Figure 7 shows the noise reduction device 100 of the present disclosure being mounted to a respiratory apparatus 162. The body 104 is mounted to one side of the respiratory apparatus 162 by the tabs 160 (see Figure 5 at 160). The tabs 150 (see Figure 4 at 150) on the cover 102 are oriented to be slidably locked with the corresponding slots 122. A seal is to be formed by the first end portion 111 and the projection 146 of the guiding member 136 to define the gas passage 138.

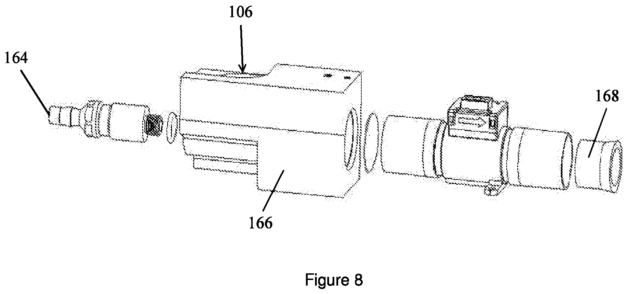

Referring to Figure 8 which shows an embodiment of the components of the respiratory apparatus (see Figure 7 at 162) , it may further include a pressurized gas inlet 164 for supplying a pressurized gas, a chamber 166 in fluid communication with the gas outlet 106 (see Figure 7 at 106) of the noise reduction device (see Figure 5 at 100) and the pressurized gas inlet 164 wherein the chamber 166 is for mixing atmospheric air and the pressurized gas, and a noise-damping device 168 disposed downstream of the chamber 166. The noise-damping device 168 is made of a porous material, for example, porous ceramic, porous plastics or porous polymeric foams, for absorbing noise in order to further minimize the noise generated when supplying a gas source to the respiratory apparatus.

It is believed that the noise reduction device 100 in the present disclosure can provide obvious noise reduction effect by decreasing the turbulence and resistance of gas flow to a larger extent and thus reducing the noise caused by the friction between the fluctuated gas flow and the gas inlet of the respiratory apparatus. Moreover, the configuration of the guiding member 136 being located on the cover 102 provides an easier and more convenient way to clean the gas passage 138. The cover 102 can be disengaged from the body 104 and subject to common sterilization methods of medical equipment. Such arrangement also facilitates replacement of the cover 102 in case abrasion or damage is found on the guiding member 136 which may increase turbulent flow of the incoming gas and thus causes noise.

It should be understood that the above only illustrates and describes examples whereby the present invention may be carried out, and that modifications and/or alterations may be made thereto without departing from the scope of the invention.

## Claims

1. A noise reduction device (100) for a respiratory apparatus (162), comprising:
a body (104) configured to be mounted on the respiratory apparatus (162), the body (104) comprising a side wall (110) and a gas outlet (106); and
a cover (102) configured to be detachably engageable with the body (104) for forming a gas inlet (156) and a gas passage (138), wherein the cover (102) comprises a guiding member (136) defining at least a part of the gas passage (138), wherein the guiding member (136) is configured to be coupled with the side wall of the body (104) to form the gas passage (138) between the body (104) and the cover (102), and wherein the gas passage (138) formed directs a flow of gas to an angular rotation about a centre of the gas outlet (106) of at least 180 degrees to 330 degrees relative to the gas inlet (156) before discharging at the gas outlet (106);
**characterized in that** the noise reduction device (100) further comprises:
a locking mechanism (152) for slidably locking the cover (102) to the body (104), wherein the locking mechanism (152) comprises at least two corresponding tabs (150) arranged on the cover (102), and at least two slots (122) respectively arranged on substantially diametrically opposite sides of the side wall (110) for complementary slide locking with the corresponding tabs on the cover (102),
and wherein the cover (102) is engaged with the body (104) to form the gas inlet (156) and the gas passage (138) by that the at least two corresponding tabs (150) arranged on the cover (102) are slidably locked to the at least two slots (122) arranged on the body (104) respectively.

2. The noise reduction device (100) of claim 1, wherein the gas passage (138) formed directs a flow of gas to an angular rotation about a centre of the gas outlet (106) of at least 270 degrees to 330 degrees relative to the gas inlet (156) before discharging at the gas outlet (106).

3. The noise reduction device (100) of claim 1, wherein the gas passage (138) formed directs a flow of gas to an angular rotation about a centre of the gas outlet (106) of at least 330 degrees relative to the gas inlet (156) before discharging at the gas outlet (106).

4. The noise reduction device (100) of claim 1, wherein the guiding member (136) is configured to extend perpendicularly from an inner surface of the cover (102) towards the body (104);
wherein the guiding member (136) is in form of a C-shape; and
wherein the guiding member (136) comprises an end portion forming a seal with a side wall of the body (104).

5. The noise reduction device (100) of claim 1, wherein a portion of the gas inlet (156) is formed between the side wall of the body (104) and a side wall of the cover (102); or,
wherein a portion of the gas inlet (156) is formed between the side wall of the cover (102) and the guiding member (136).

6. The noise reduction device (100) of claim 1, wherein the cover (102) has a flow deflecting portion (148) adjacent to the gas inlet (156).

7. The noise reduction device (100) of claim 1, wherein the body (104) is arranged to house a filter (115) therein.

8. The noise reduction device (100) of claim 7,
wherein inner surface of the body (104) has a plurality of upright protrusions (118) to support the filter; and
wherein the filter (115) is sandwiched between the guiding member (136) and the body (104).

9. The noise reduction device (100) of claim 1, wherein area enclosed by the guiding member (136) is at least twice than area of the gas outlet (106).

10. The noise reduction device (100) of claim 1, wherein the gas inlet (156) is arranged perpendicularly to the gas outlet (106).

11. The noise reduction device (100) of claim 1, wherein the gas inlet (156) is arranged for supplying atmospheric air to the respiratory apparatus (162).

12. A respiratory apparatus (162) comprising a noise reduction device (100) according to any one of claims 1 to 11.

13. The respiratory apparatus (162) of claim 12, further comprising: a pressurized gas inlet (156) for supplying a pressurized gas; a chamber (166) in fluid communication with the gas outlet (106) of the noise reduction device (100) and the pressurized gas inlet (156), the chamber (166) for mixing atmospheric air and the pressurized gas; and a noise-damping device (168) disposed downstream of the chamber (166).

## Patentansprüche

1. Geräuschreduktionsvorrichtung (100) für eine Atmungsvorrichtung (162), umfassend:
einen Körper (104), der konfiguriert ist, um an der Atmungsvorrichtung (162) montiert zu werden, wobei der Körper (104) eine Seitenwand (110) und einen Gasauslass (106) umfasst; und
eine Abdeckung (102), die konfiguriert ist, um lösbar mit dem Körper (104) in Eingriff gebracht zu werden, um einen Gaseinlass (156) und einen Gasdurchgang (138) zu bilden, wobei die Abdeckung (102) ein Führungselement (136) umfasst, das zumindest einen Teil des Gasdurchgangs (138) definiert, wobei das Führungselement (136) konfiguriert ist, um mit der Seitenwand des Körpers (104) gekoppelt zu werden, um den Gasdurchgang (138) zwischen dem Körper (104) und der Abdeckung (102) zu bilden, und wobei der gebildete Gasdurchgang (138) einen Gasstrom zu einer Winkeldrehung um eine Mitte des Gasauslasses (106) um mindestens 180 Grad bis 330 Grad relativ zu dem Gaseinlass (156) vor Entladen an dem Gasauslass (106) leitet;
dass die Geräuschreduktionsvorrichtung (100) ferner Folgendes umfasst:
einen Verriegelungsmechanismus (152) zum gleitbaren Verriegeln der Abdeckung (102) an dem Körper (104), wobei der Verriegelungsmechanismus (152) mindestens zwei entsprechende Laschen (150), die an der Abdeckung (102) angeordnet sind, und mindestens zwei Schlitze (122), die jeweils an im Wesentlichen diametral gegenüberliegenden Seiten der Seitenwand (110) angeordnet sind, um eine komplementäre Gleitverriegelung mit den entsprechenden Laschen (120) an der Abdeckung (102) zu bilden, umfasst, und wobei die Abdeckung (102) mit dem Körper (104) in Eingriff steht, um den Gaseinlass (156) bzw. den Gasdurchgang (138) zu bilden, indem die mindestens zwei entsprechenden Laschen (150), die an der Abdeckung (102) angeordnet sind, gleitbar mit den mindestens zwei Schlitzen (122) verriegelt sind, die jeweils an dem Körper (104) angeordnet sind.

2. Geräuschreduktionsvorrichtung (100) nach Anspruch 1, wobei der gebildete Gasdurchgang (138) einen Gasstrom zu einer Winkeldrehung um eine Mitte des Gasauslasses (106) um mindestens 270 Grad bis 330 Grad relativ zu dem Gaseinlass (156) vor Entladen am Gasauslass (106) leitet.

3. Geräuschreduktionsvorrichtung (100) nach Anspruch 1, wobei der gebildete Gasdurchgang (138) einen Gasstrom zu einer Winkeldrehung um eine Mitte des Gasauslasses (106) um mindestens 330 Grad relativ zu dem Gaseinlass (156) vor Entladen am Gasauslass (106) leitet.

4. Geräuschreduktionsvorrichtung (100) nach Anspruch 1, wobei das Führungselement (136) konfiguriert ist, sich senkrecht von einer Innenfläche der Abdeckung (102) in Richtung des Körpers (104) zu erstrecken;
wobei das Führungselement (136) in Form einer C-Form ausgebildet ist; und
wobei das Führungselement (136) einen Endabschnitt umfasst, der eine Dichtung mit einer Seitenwand des Körpers (104) bildet.

5. Geräuschreduktionsvorrichtung (100) nach Anspruch 1, wobei ein Abschnitt des Gaseinlasses (156) zwischen der Seitenwand des Körpers (104) und einer Seitenwand der Abdeckung (102) ausgebildet ist; oder
wobei ein Abschnitt des Gaseinlasses (156) zwischen der Seitenwand der Abdeckung (102) und dem Führungselement (136) ausgebildet ist.

6. Geräuschreduktionsvorrichtung (100) nach Anspruch 1, wobei die Abdeckung (102) einen Strömungsablenkabschnitt (148) benachbart zu dem Gaseinlass (156) aufweist.

7. Geräuschreduktionsvorrichtung (100) nach Anspruch 1, wobei der Körper (104) angeordnet ist, um einen Filter (115) darin aufzunehmen.

8. Geräuschreduktionsvorrichtung (100) nach Anspruch 7, wobei die Innenfläche des Körpers (104) eine Vielzahl von aufrechten Vorsprüngen (118) zum Stützen des Filters aufweist; und
wobei der Filter (115) sandwichartig zwischen dem Führungselement (136) und dem Körper (104) angeordnet ist.

9. Geräuschreduktionsvorrichtung (100) nach Anspruch 1, wobei ein von dem Führungselement (136) umschlossener Bereich mindestens doppelt so groß ist wie die Fläche des Gasauslasses (106).

10. Geräuschreduktionsvorrichtung (100) nach Anspruch 1, wobei der Gaseinlass (156) senkrecht zum Gasauslass (106) angeordnet ist.

11. Geräuschreduktionsvorrichtung (100) nach Anspruch 1, wobei der Gaseinlass (156) zum Zuführen von atmosphärischer Luft zu der Atmungsvorrichtung (162) angeordnet ist.

12. Atmungsvorrichtung (162), umfassend eine Geräuschreduktionsvorrichtung (100) nach einem der Ansprüche 1 bis 11.

13. Atmungsvorrichtung (162) nach Anspruch 12, ferner umfassend: einen Druckgaseinlass (156) zum Zuführen eines unter Druck stehenden Gases; eine Kammer (166) in Fluidverbindung mit dem Gasauslass (106) der Geräuschreduktionsvorrichtung (100) und dem Druckgaseinlass (156), wobei die Kammer (166) zum Mischen atmosphärischer Luft und des unter Druck stehenden Gases angeordnet ist; und eine Geräuschdämpfungsvorrichtung (168), die stromabwärts der Kammer (166) angeordnet ist.

## Revendications

1. Dispositif de réduction de bruit (100) pour un appareil respiratoire (162), comprenant :
un corps (104) configuré pour être monté sur l'appareil respiratoire (162), le corps (104) comprenant une paroi latérale (110) et une sortie de gaz (106) ; et
un couvercle (102) configuré pour s'enclencher de manière amovible avec le corps (104) pour former une entrée de gaz (156) et un passage de gaz (138), dans lequel le couvercle (102) comprend un élément de guidage (136) délimitant au moins une partie du passage de gaz (138), dans lequel l'élément de guidage (136) est configuré pour être couplé à la paroi latérale du corps (104) pour former le passage de gaz (138) entre le corps (104) et le couvercle (102), et dans lequel le passage de gaz (138) formé dirige un flux de gaz vers une rotation angulaire autour d'un centre de la sortie de gaz (106) d'au moins 180 degrés à 330 degrés par rapport à l'entrée de gaz (156) avant de se décharger à la sortie de gaz (106) ;
**caractérisé en ce que** le dispositif de réduction de bruit (100) comprend en outre :
un mécanisme de verrouillage (152) pour verrouiller de manière coulissante le couvercle (102) sur le corps (104), dans lequel le mécanisme de verrouillage (152) comprend au moins deux languettes correspondantes (150) disposées sur le couvercle (102), et au moins deux fentes (122) disposées respectivement sur des côtés sensiblement opposés dans la direction de diamètre de la paroi latérale (110) pour verrouiller de manière coulissante et complémentaire avec les languettes correspondantes (120) sur le couvercle (102), et dans lequel le couvercle (102) est enclenché avec le corps (104) pour former l'entrée de gaz (156) et le passage de gaz (138) de sorte que les au moins deux languettes correspondantes (150) disposées sur le couvercle (102) sont verrouillées de manière coulissante sur les au moins deux fentes (122) disposées sur le corps (104) respectivement.

2. Dispositif de réduction de bruit (100) selon la revendication 1, dans lequel le passage de gaz (138) formé dirige un flux de gaz vers une rotation angulaire autour d'un centre de la sortie de gaz (106) d'au moins 270 degrés à 330 degrés par rapport à l'entrée de gaz (156) avant de se décharger à la sortie de gaz (106).

3. Dispositif de réduction de bruit (100) selon la revendication 1, dans lequel le passage de gaz (138) formé dirige un flux de gaz vers une rotation angulaire autour d'un centre de la sortie de gaz (106) d'au moins 330 degrés par rapport à l'entrée de gaz (156) avant de se décharger à la sortie de gaz (106).

4. Dispositif de réduction de bruit (100) selon la revendication 1, dans lequel l'élément de guidage (136) est configuré pour s'étendre perpendiculairement à partir d'une surface intérieure du couvercle (102) vers le corps (104) ;
dans lequel l'élément de guidage (136) présente une forme C ; et
dans lequel l'élément de guidage (136) comprend une partie d'extrémité formant un joint avec une paroi latérale du corps (104).

5. Dispositif de réduction de bruit (100) selon la revendication 1, dans lequel une partie de l'entrée de gaz (156) est formée entre la paroi latérale du corps (104) et une paroi latérale du couvercle (102) ; ou
dans lequel une partie de l'entrée de gaz (156) est formée entre la paroi latérale du couvercle (102) et l'élément de guidage (136).

6. Dispositif de réduction de bruit (100) selon la revendication 1, dans lequel le couvercle (102) comporte une partie de déviation de flux (148) adjacente à l'entrée de gaz (156).

7. Dispositif de réduction de bruit (100) selon la revendication 1, dans lequel le corps (104) est disposé dans lequel est logé un filtre (115).

8. Dispositif de réduction de bruit (100) selon la revendication 7, dans lequel la surface intérieure du corps (104) comporte une pluralité de saillies verticales (118) pour soutenir le filtre ; et
dans lequel le filtre (115) est disposé en sandwich entre l'élément de guidage (136) et le corps (104).

9. Dispositif de réduction de bruit (100) selon la revendication 1, dans lequel une surface délimitée par l'élément de guidage (136) est au moins deux fois la surface de la sortie de gaz (106).

10. Dispositif de réduction de bruit (100) selon la revendication 1, dans lequel l'entrée de gaz (156) est disposée perpendiculairement à la sortie de gaz (106).

11. Dispositif de réduction de bruit (100) selon la revendication 1, dans lequel l'entrée de gaz (156) est disposée pour alimenter l'appareil respiratoire (162) en air atmosphérique.

12. Appareil respiratoire (162) comprenant un dispositif de réduction de bruit (100) selon l'une quelconque des revendications 1 à 11.

13. Appareil respiratoire (162) selon la revendication 12, comprenant en outre : une entrée de gaz sous pression (156) pour alimenter un gaz sous pression ; une chambre (166) en communication fluide avec la sortie de gaz (106) du dispositif de réduction de bruit (100) et l'entrée de gaz sous pression (156), la chambre (166) étant utilisée pour mélanger l'air atmosphérique et le gaz sous pression ; et un dispositif d'insonorisation (168) disposé en aval de la chambre (166).
